# EUROPÄISCHE PATENTANMELDUNG

(11) **EP 2 368 516 A2**
(43) Veröffentlichungstag der Anmeldung: **28.09.2011**
(21) Anmeldenummer: 11158117.9
(22) Anmeldetag: 14.03.2011
(51) Int. Cl.: A61C 1/08

(54) **Hülsengeführtes Werkzeugsystem**

(30) Priorität: 25.03.2010 DE 102010012960; 21.04.2010 DE 102010018410
(71) Anmelder: Schneider, Robert, 73491 Neuler (DE); Kiener, Hannelore, 73466 Lauchheim (DE)
(72) Erfinder: Schneider, Robert, 73491 Neuler (DE); Kiener, Hannelore, 73466 Lauchheim (DE)
(74) Vertreter: Ganahl, Bernhard

(57) **Zusammenfassung**

Die Erfindung betrifft ein hülsengeführtes Werkzeugsystem zum Präparieren eines Implantatbettes (13) zum anschließenden Setzen eines Implantates mit einer Setzhülse (12), welche in eine Bohrschablone (14) befestigbar ist, die zur Implantatsetzung im Mund des Patienten eingesetzt wird, wobei die Setzhülse (12) an der Stelle der Implantatsetzung in der Bohrschablone (14) positionierbar ist, wobei eine Werkzeugeinheit (16) in die Setzhülse (12) einsetzbar ist, welche zumindest aus einem Werkzeug (18) und einer Führungshülse (17) besteht und die Führungshülse (17) das Werkzeug (18) längsverschiebbar aufnimmt und daran befestigt ist oder eine Führungshülse (17), welche zur Aufnahme und Führung eines Werkzeuges (18) vorgesehen ist, in die Setzhülse (12) einsetzbar und lagegesichert zu dieser verriegelbar ist sowie anschließend das Werkzeug (18) längsverschiebbar geführt aufnimmt.

## Beschreibung

Die Erfindung betrifft ein hülsengeführtes Werkzeugsystem zum Präparieren eines Implantatbettes zum anschließenden Setzen eines Implantates.

Zum Präparieren eines Implantatbettes für das anschließende Setzen eines Implantates sind bereits ein Verfahren sowie ein hülsengeführtes Werkzeugsystem bekannt, bei dem zunächst ein Mastermodell gefertigt wird, aus welchem die Patientensituation bezüglich der Lage und Anzahl der Zähne hervorgeht. Dieses Mastermodell ist die Grundlage für die Herstellung einer sogenannten Scanschablone. Diese Scanschablone wird mit Hilfe von Fixierelementen an dem Mastermodell fixiert, um anschließend die Verbindung einer digitalen Implantatplanung mit der Bohrschablonenherstellung zu gewährleisten. Der Patient wird mit eingesetzter Scanschablone mit einem 3D-Scanner gescannt. Aus diesem Scanvorgang werden computerunterstützt Daten generiert, aus denen unter Berücksichtigung der Patientenanatomie und des gewünschten prothetischen Ergebnisses die Position der Implantate ermittelt und festgelegt wird. Anschließend wird bevorzugt die Scanschablone in eine Bohrschablone umgearbeitet, indem an der ermittelten Position zum Setzen eines Implantats eine Bohrhülse beziehungsweise eine Setzhülse in die Scanschablone eingearbeitet wird, so dass die Scanschablone zur Bohrschablone umgearbeitet ist. Ebenso kann separat auch eine Bohrschablone mit einer entsprechenden Setzhülse hergestellt werden. Anschließend wird die Bohrschablone im Mund des Patienten positioniert. Zur Führung eines Werkzeuges, wie beispielsweise einem Bohrer, wird ein sogenannter Löffel verwendet, der an dem am Handgriff gegenüberliegenden Ende eine randoffene Hülse aufweist, so dass das Werkzeug darin einsetzbar und längsverschiebbar geführt ist. Anschließend wird der Löffel mit dem in der randoffenen Hülse geführten Werkzeug in die Setzhülse eingesetzt. In einem nächsten Arbeitsschritt wird das Werkzeug durch den Operateur auf das Implantatbett zugeführt und dieses für die geplante Setzung des Implantates präpariert. Dabei muss mit der zweiten Hand der Löffel in einer exakten Position gehalten werden. Für die Präparation können mehrere Arbeitsschritte erforderlich sein, die ein nochmaliges Entfernen und Wiedereinsetzen verschiedener Werkzeuge erfordert.

Ein solches hülsengeführtes Werkzeugsystem für ein schablonengeführtes Implantieren weist den Nachteil auf, dass der Operateur gleichzeitig mit beiden Händen den Patienten behandeln muss. Aufgrund der Positionierung des Werkzeuges über den Löffel kann es leicht zu Ungenauigkeiten kommen, da über den Löffel eine große Hebelkraft auf die Setzhülse bei einer bereits leichten Verkippung wirkt.

Der Erfindung liegt deshalb die Aufgabe zugrunde, ein hülsengeführtes Werkzeugsystem zum Präparieren eines Implantatbettes zum anschließenden Setzen eines Implantates vorzuschlagen, welches eine einfache Handhabung und eine präzise Präparation des Implantatbettes ermöglicht.

Diese Aufgabe wird durch die Merkmale des Anspruchs 1 gelöst. Weitere vorteilhafte Ausgestaltungen und Weiterbildungen sind in den weiteren abhängigen Ansprüchen angegeben.

Gemäß der ersten erfindungsgemäßen Ausführungsform ist eine Werkzeugeinheit in die Setzhülse der Bohrschablone einsetzbar, welche zumindest aus einem Werkzeug und einer Führungshülse besteht und die Führungshülse das Werkzeug längsverschiebbar aufnimmt, wobei die Führungshülse an dem Werkzeug befestigt ist. Diese Ausführungsform weist den Vorteil auf, dass eine Werkzeugeinheit bereitgestellt ist, welche im Einsatzfall in die Setzhülse eingesetzt wird und sich anschließend selbständig hält sowie das Werkzeug zur Setzhülse zentriert. Dadurch wird ein einhändiges Arbeiten möglich, da lediglich eine Zuführbewegung des Bohrers in das Implantatbett zum Präparieren erforderlich und somit auch ein einfacheres und schnelleres Handling ermöglicht. Ein Verwinden der Bohrschablone über den Hebel des Löffels ist nicht gegeben.

Eine alternative Ausführungsform der Erfindung sieht vor, dass eine Führungshülse in die Setzhülse einsetzbar ist, welche während oder nach dem Einsetzen lagegesichert zur Setzhülse verriegelbar und zur Führung des Werkzeuges ausgebildet ist. Diese Anordnung ermöglicht dieselben Vorteile wie die erste erfindungsgemäße Ausführungsform. Ebenfalls ist ein einhändiges Arbeiten möglich, da die Führungshülse lagegesichert in der Setzhülse fixiert und durch die Führungshülse ein exaktes Führen des Werkzeuges gegeben ist.

Nach einer bevorzugten Ausgestaltung der Erfindung ist vorgesehen, dass die Führungshülse am Schaft des Werkzeuges angreift. Dadurch ist eine leichtgängige Führung des Werkzeuges in der Führungshülse gegeben. Bei bekannten Systemen ist das Werkzeug hingegen über die Schneiden geführt, was zu einem Beschädigen der Setzhülse oder einem Verkanten der Schneiden führen kann.

Des Weiteren ist bevorzugt vorgesehen, dass das Werkzeug einen gegenüber einem Durchmesser des Schaftes größeren Werkzeugkopf aufweist und entfernt zum Werkzeugkopf ein lösbares Sicherungselement am Schaft aufnimmt, wobei dazwischen liegend die Führungshülse angeordnet ist. Diese Ausführungsform weist den Vorteil auf, dass durch das am Schaft angreifende und angeordnete Sicherungselement ein Tiefenanschlag des Werkzeuges gegeben ist. Beispielsweise kann dies beim Einsatz eines Spreizwerkzeuges oder eines Bohrwerkzeuges beziehungsweise Fräswerkzeuges als auch eines Spaltwerkzeuges oder eines Stanzwerkzeuges beziehungsweise einem Hohlbohrwerkzeug von Vorteil sein. Das Sicherungselement ist insbesondere für Reinigungs- und Desinfektionszwecke lösbar, so dass die Führungshülse vom Schaft entfernt werden kann.

Des Weiteren ist bevorzugt vorgesehen, dass zwischen dem Werkzeugkopf und dem Sicherungselement die Führungshülse und eine Federführung mit zumindest einem Federelement angeordnet sind, wobei die Federführung zwischen der Führungshülse und dem Sicherungselement angeordnet ist. Diese Anordnung kann insbesondere beim Einsatz von Bohr- oder Fräswerkzeugen vorgesehen sein. Dadurch kann einerseits ein Tiefenanschlag gegeben sein und andererseits nach dem Ende der Zustellbewegung ein selbständiges Abheben des Werkzeuges aus dem Bohrgrund heraus sichergestellt werden.

Des Weiteren sind an dem Schaft des Werkzeuges bevorzugt mehrere im Abstand zueinander angeordnete Aufnahmevertiefungen für das lösbare Sicherungselement vorgesehen. Dadurch kann das Werkzeug bezüglich dessen Eintauchtiefe in das Implantatbett vorbestimmt und begrenzt werden. Dies erleichtert die Tätigkeit des Operateurs.

Zwischen dem Werkzeugkopf und dem lösbaren Sicherungselement ist bevorzugt zumindest eine Trennstelle vorgesehen. Beispielsweise kann bei einem Bohr- oder Fräswerkzeug am Schaft zwischen dem Werkzeugkopf und dem Sicherungselement eine Trennstelle vorgesehen sein, wodurch ein einfaches Zerlegen der Werkzeugeinheit für die Reinigung und Desinfektion ermöglicht wird. Analoges gilt auch für weitere zum Einsatz gelangende Werkzeuge, wie beispielsweise einem Spreizwerkzeug, einem Spaltwerkzeug oder einem Stanzwerkzeug beziehungsweise einem Hohlbohrwerkzeug.

Eine weitere Ausführungsform der Erfindung sieht vor, dass an einem dem Werkzeugkopf gegenüberliegenden Ende oder an einem dem Werkzeugkopf gegenüberliegenden Ende des Schaftes des Werkzeuges eine Werkzeugaufnahme lösbar befestigt ist und eine lösbare Trennstelle bildet. Diese alternative Ausführungsform ermöglicht ebenso ein leichtes und einfaches Zerlegen der Werkzeugeinheit, so dass eine dazwischen angeordnete Führungshülse für den Schaft des Werkzeuges zur Reinigung in einfacher Weise entnommen werden kann.

Eine bevorzugte Ausgestaltung sieht vor, dass die lösbare Trennstelle als Verschraubung ausgebildet ist. Diese Verschraubung ist dabei vorzugsweise in axialer Richtung des Schaftes ausgerichtet und kann zwei Schaftabschnitte oder einen Werkzeugkopf mit einem Schaft miteinander verbinden. Eine solche Verschraubung ist insbesondere bei noch hinreichend großen Durchmessern von Vorteil und stellt eine einfache Lösung dar. Eine alternative Ausgestaltung der lösbaren Trennstelle sieht vor, dass durch einen thermischen Aufschrumpfprozess zwischen den beiden zu verbindenden Teilen, wie beispielsweise Werkzeugkopf/Schaft, Schaft/Schaft oder Schaft/Werkzeugaufnahme eine lösbare Verbindung geschaffen wird. Beispielsweise können eine zylindrische Bohrung in der Werkzeugaufnahme und ein konisches Ende des Schaftes vorgesehen sein, die durch Aufschrumpfen miteinander verbunden werden. Ebenso kann eine Vertauschung vorgesehen sein. Des Weiteren können zwei konische Abschnitte mit unterschiedlicher Steigung durch Aufschrumpfung zueinander befestigt werden. Des Weiteren kann als lösbare Trennstelle vorteilhafterweise eine Verdrehsicherung zwischen den zwei zu verbindenden Bauteilen und ein zusätzlicher Halte- oder Sicherungsstift zur axialen Fixierung der Teile zueinander vorgesehen sein. Die Auswahl der Ausgestaltung für eine lösbare Trennstelle steht auch in Abhängigkeit der zur Verfügung stehenden Durchmesser des Schaftes des Werkzeuges.

Bei der vorteilhaften Ausgestaltung der lösbaren Trennstelle mit einer Werkzeugaufnahme ist bevorzugt eine Verdrehsicherung in der Werkzeugaufnahme vorgesehen und eine Bohrung für einen einbringbaren Haltestift, insbesondere Gewindestift, eingebracht, der zur axialen Fixierung des Schaftendes zur Werkzeugaufnahme dient. Diese Werkzeugaufnahme umgibt bevorzugt das Schaftende. Dadurch wird ein größerer Umfangsabschnitt der Werkzeugaufnahme geschaffen, wodurch das Einbringen eines Haltestiftes oder Gewindestiftes beziehungsweise Sicherungsstiftes vereinfacht ist. Bevorzugt kann die Verdrehsicherung, die in einer Sacklochbohrung der Werkzeugaufnahme vorgesehen sein kann, durch Erodieren hergestellt werden.

Eine weitere bevorzugte Ausgestaltung der Trennstelle durch eine lösbare Werkzeugaufnahme sieht vor, dass das zum Werkzeugkopf weisende Ende der Werkzeugaufnahme ein Führungselement und vorzugsweise die Führungshülse eine Federführung aufweist, so dass nach dem Verbinden der Werkzeugaufnahme mit dem Schaft des Werkzeuges eine federgelagerte Anordnung des Werkzeuges zur Setzhülse gegeben ist, die bewirkt, dass nach der Zuführbewegung des Werkzeuges in das Implantatbett ein selbständiges Abheben des Werkzeuges erfolgt. Die Federführung ist bevorzugt einteilig an der Führungshülse und der Werkzeugaufnahme integriert.

Des Weiteren ist die lagegesicherte Führungshülse vorteilhafterweise zur Aufnahme eines Spaltwerkzeuges, Bohr- oder Fräswerkzeuges vorgesehen, welche insbesondere mit einem Schlitzverschluss zur Setzhülse gesichert ist. Eine solche Anordnung weist den Vorteil auf, dass ein schablonengeführtes Spreizen, Bohren oder Fräsen möglich ist. Dabei wird bevorzugt ein sogenanntes Piezowerkzeug zur Spaltung des Implantatbettes beziehungsweise des Kiefers eingesetzt. Durch die rotationsgesicherte Anordnung der Führungshülse zur Setzhülse als auch dem Schlitzverschluss und des vorzugsweise ausgerichteten Längsschlitzes in der Führungshülse kann das Spaltwerkzeug durch die Ausrichtung des Werkzeuges während dem Spalt- oder Schlitzprozess des Kiefers aufrechterhalten bleiben. Durch diese Führungshülse kann auch in Abhängigkeit der Kontur des Spaltes in der Führungshülse eine geführte Langlochfräsung, eine Konturfräsung oder dergleichen durchgeführt werden.

Eine weitere bevorzugte Ausgestaltung der Erfindung sieht vor, dass die Führungshülse einen radial gegenüber einer an der Setzhülse angreifenden Umfangsfläche hervorstehenden Verriegelungszapfen aufweist, welcher in eine Kulisse der Setzhülse einsetzbar ist. Dadurch kann eine einfach zu handhabende Lagesicherung und Verriegelung der Führungshülse zur Setzhülse erfolgen. Die Führungshülse kann dabei verschieden ausgebildete Werkzeugführungen im Inneren aufweisen. Beispielsweise kann eine gegenüber einer Längsachse und der Setzhülse und der Führungshülse geneigte Bohrung vorgesehen sein, um eine geneigte Werkzeugführung zu ermöglichen. Ebenso können beliebige Spaltkonturen in der Führungshülse vorgesehen sein.

Eine vorteilhafte Weiterbildung der Führungshülse, welche lagegesichert in der Setzhülse anordenbar ist, umfasst an einem zum Implantatbett weisenden Ende eine Tiefenbegrenzung, insbesondere einen Sicherungsring. Dadurch kann in einfacher Weise die Zustellbewegung eines Werkzeuges begrenzt werden, welches bevorzugt zu dessen Wegbegrenzung einen Ringbund oder Absatz am Schaft aufweist.

Beim hülsengeführten Werkzeugsystem kommen bevorzugt Werkzeuge, wie beispielsweise Bohrer, Fräser, Stanzwerkzeuge oder Spreizwerkzeuge, zum Einsatz.

Insbesondere Bohr- oder Fräswerkzeuge können innenliegende Kühlmittelbohrungen aufweisen, so dass eine gekühlte Bearbeitung des Kiefers ermöglicht ist und zusätzliche Beschädigungen verhindert werden.

Die Erfindung sowie weitere vorteilhafte Ausführungsformen und Weiterbildungen derselben werden im Folgenden anhand der in den Zeichnungen dargestellten Beispiele näher beschrieben und erläutert. Die der Beschreibung und den Zeichnungen zu entnehmenden Merkmale können einzeln für sich oder zu mehreren in beliebiger Kombination erfindungsgemäß angewandt werden. Es zeigen:
- Figur 1: eine schematische Schnittdarstellung einer ersten Ausführungsform des erfindungsgemäßen Werkzeugsystems,
- Figur 2a: eine schematische Schnittansicht einer alternativen Ausführungsform zu Figur 1,
- Figur 2b: eine schematische Schnittansicht einer alternativen Ausführungsform zu Figur 2a,
- Figur 3a: eine schematische Schnittansicht einer weiteren alternativen Ausführungsform zu Figur 1,
- Figur 3b: eine schematische Schnittansicht einer weiteren alternativen Ausführungsform zu Figur 3a,
- Figuren 4a und b: eine schematische Schnittansicht und Draufsicht auf eine weitere alternative Ausführungsform zu Figur 1,
- Figur 5a: eine schematische Schnittansicht von einer weiteren alternativen Ausführungsform,
- Figur 5b: eine schematische Schnittansicht einer weiteren alternativen Ausführungsform zu Figur 5a,
- Figuren 6a bis c: schematische Schnittdarstellungen von erfindungsgemäßen in Setzhülsen eingesetzten Führungshülsen,
- Figur 7: eine schematische Schnittdarstellung einer weiteren alternativen Ausführungsform und
- Figuren 8a bis c: schematische Schnittdarstellungen von einer lösbaren Trennstelle zwischen einer Werk- zeugaufnahme und einem Schaft eines Werkzeuges.

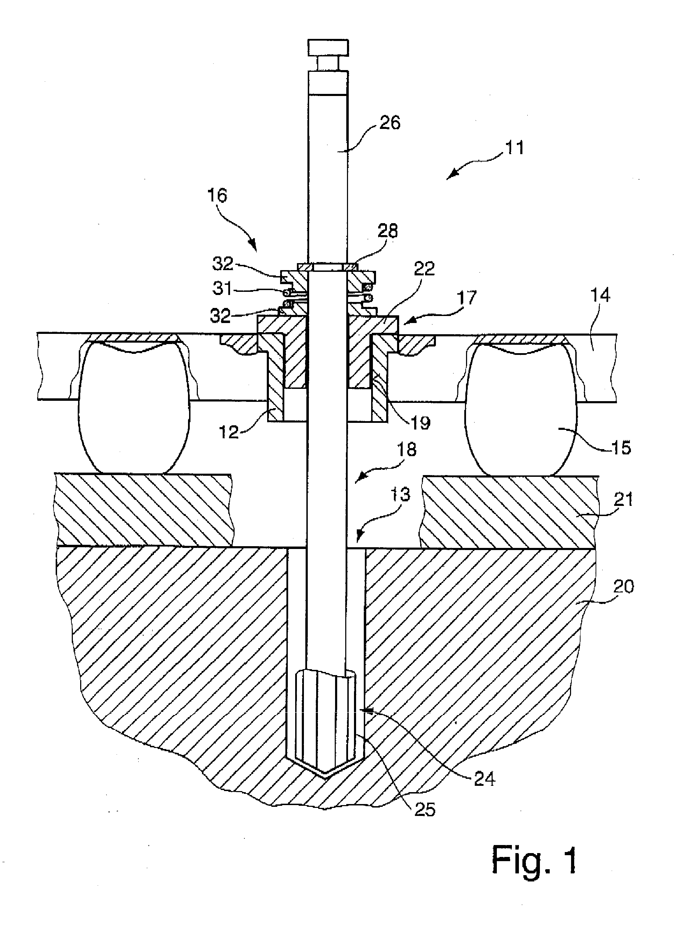

In Figur 1 ist eine schematische Schnittansicht eines erfindungsgemäßen hülsengeführten Werkzeugsystems 11 dargestellt. Dieses Werkzeugsystem 11 umfasst eine Setzhülse 12, welche in eine nur teils dargestellte Bohrschablone 14 einsetzbar ist. Solche Bohrschablonen 14 werden separat oder zumeist aus Scanschablonen durch Einarbeiten der Setzhülse 12 hergestellt. Diese Bohrschablone 12 wird vor dem Präparieren eines Implantatbettes 13 auf die Zähne 15 und/oder den Kiefer 20 des Patienten aufgesetzt. Die Setzhülse 12 nimmt eine Werkzeugeinheit 16 auf, welche eine Führungshülse 17 und ein in der Führungshülse 17 längsverschiebbar geführtes Werkzeug 18 umfasst. Die Führungshülse 17 weist eine Umfangsfläche 19 auf, durch welche die Führungshülse 17 radial zur Setzhülse 12 ausrichtet ist. Am oberen Ende des Außenumfangs 19 ist eine Schulter 22 ausgebildet, durch welche die Einsetzposition der Führungshülse 17 in die Setzhülse 12 begrenzt ist.

Das Werkzeug 18, welches beispielsweise als Bohrer oder Fräser ausgeführt ist, umfasst einen Werkzeugkopf 24, an welchem Schneiden 25 vorgesehen sind. Der Werkzeugkopf 24 wird von einem Schaft 26 getragen, der im Durchmesser kleiner als der Werkzeugkopf 24 ausgebildet ist. An dem Schaft 26 ist lösbar ein Sicherungselement 28 vorgesehen. Dieses Sicherungselement 28 kann beispielsweise ein Sicherungsring sein, der dafür sorgt, dass die zwischen dem Sicherungselement 28 und dem Führungskopf 24 entlang des Schaftes 26 verschiebbar angeordnete Führungshülse 17 unverlierbar zum Werkzeug 18 befestigt ist und die Werkzeugeinheit 16 bildet.

Ergänzend kann die Werkzeugeinheit 16 zumindest ein Federelement 31 und eine Federführung 32 aufweisen, wobei sich die Federführung 32 zwischen der Führungshülse 17 und dem Sicherungselement 28 abstützt. Dadurch wird durch das als Druckfeder ausgebildete Federelement 31 das Werkzeug 18 immer in seine obere Lage nach dem Betätigen bewegt.

Eine nicht näher dargestellte Ausführungsform der Werkzeugeinheit 16 kann ein Werkzeug 18 umfassen, welches innenliegende Kühlkanäle oder Kühlbohrungen aufweist, so dass während dem Bohren Kühlwasser oder ein Kühlmedium zugeführt werden kann.

Sobald die Bearbeitung der in die Setzhülse 12 eingesetzten Werkzeugeinheit 16 beendet ist, kann diese in einfacher Weise und komplett aus der Setzhülse 12 herausgeführt werden, so dass anschließend eine weitere Werkzeugeinheit 16 beispielsweise mit einem größeren oder längeren Bohrkopf eingesetzt werden kann. Dieses erfindungsgemäße Werkzeugsystem 11 ermöglicht somit ein schnelles und exaktes Arbeiten. Darüber hinaus ist eine einhändige Bearbeitung möglich, da die Werkzeugeinheit 16 durch die Führungshülse 17 selbständig in der Setzhülse 12 ausgerichtet und geführt als auch positioniert ist.

Nach dem Gebrauch der Werkzeugeinheit 16 kann durch das Entfernen des Sicherungselementes 28 in einfacher Weise eine Zerlegung in die einzelnen Komponenten erfolgen, um die Reinigung und Desinfektion durchzuführen.

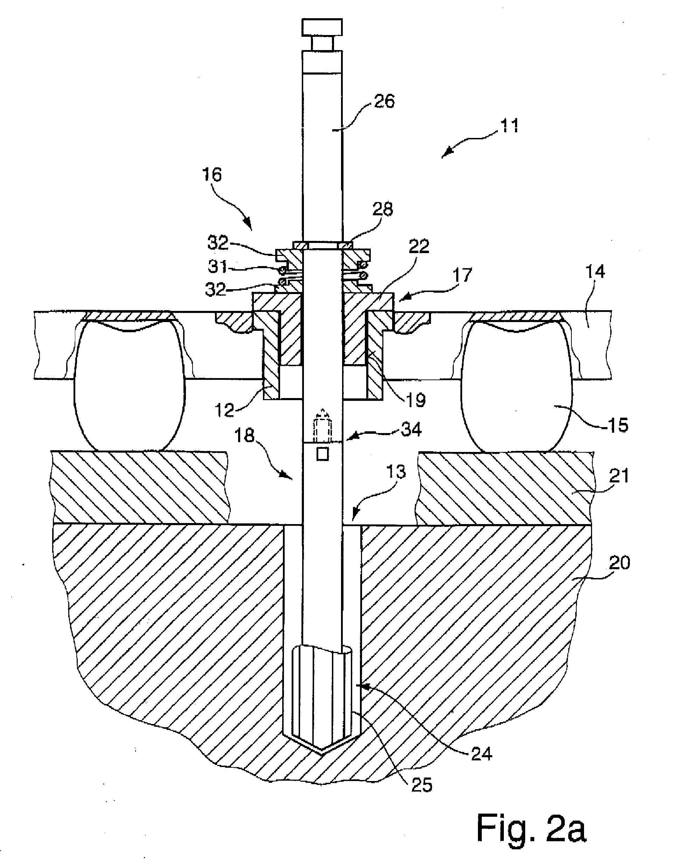

In Figur 2a ist eine schematische Schnittdarstellung einer alternativen Ausführungsform zu Figur 1 dargestellt. Im Hinblick auf die Übereinstimmungen wird vollständig auf Figur 1 Bezug genommen. Abweichend zu Figur 1 weist diese Werkzeugeinheit 16 gemäß Figur 2a ein Werkzeug 18 mit einem Schaft 26 auf, der zumindest eine Trennstelle 34 umfasst. Beispielsweise ist ein Teil des Schaftes 26 mit dem Werkzeugkopf 24 über ein Schraubgewinde mit dem weiteren Teil des Schaftes 26 verbunden, an dem das Sicherungselement 28 befestigt ist. Diese Ausführungsform weist den Vorteil auf, dass zur Reinigung und Desinfektion ein Lösen des Sicherungselementes 28 von dem Schaft 26 nicht erforderlich ist, sondern durch die Lösung des Schraubgewindes eine einfache Zerlegung der Werkzeugeinheit 16 in deren Komponenten ermöglicht ist.

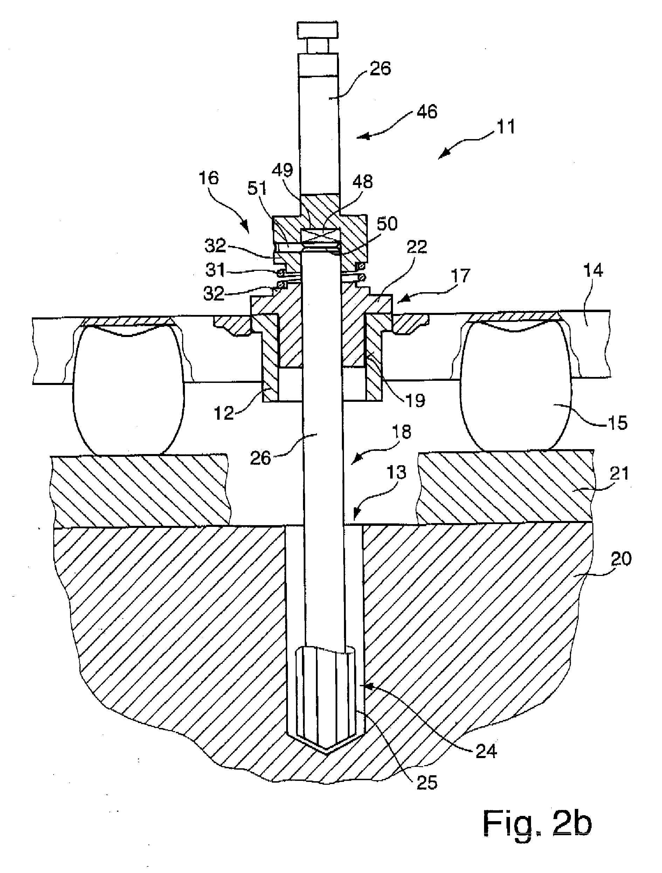

In Figur 2b ist eine alternative Ausführungsform zu Figur 2a dargestellt. Gemäß dieser Ausführungsform besteht die Werkzeugeinheit 16 aus einem Werkzeug 18, einer Werkzeugaufnahme 46, die lösbar am Schaft 26 des Werkzeuges 18 angeordnet ist, einer am Schaft 26 des Werkzeuges 18 anordenbaren Führungshülse 17 sowie vorzugsweise einem zwischen der Führungshülse 17 und der Werkzeugaufnahme 46 angeordneten Federelement 31. Die Werkzeugaufnahme 46 weist bevorzugt eine Vertiefung 49 oder Sacklochbohrung auf, in welche ein Ende des Schaftes 26 einsetzbar ist. Dabei weist das Ende des Schaftes 26 eine Verdrehsicherung 48 in Form einer Abflachung oder dergleichen auf, welche mit einer entsprechenden Fläche in der Vertiefung 49 der Werkzeugaufnahme 46 korrespondiert. Diese Verdrehsicherung 48 dient zur Drehmomentaufnahme sowie Übertragung und kann beispielsweise durch Erodieren beziehungsweise Senkerodieren hergestellt werden. Am oberen Ende des Schaftes 26 des Werkzeuges 18 ist bevorzugt eine Nut 50, insbesondere in Form einer V-förmigen Nut, vorgesehen, in welcher ein Haltestift 51 radial eingreift und das Ende des Schaftes 26 axial zur Werkzeugaufnahme 46 sichert. Bei diesem Haltestift 51 kann es sich um einen Gewindestift oder dergleichen handeln.

Bei dieser Ausführungsform ist die Federführung 32 bevorzugt einteilig am stirnseitigen Ende der Werkzeugaufnahme 46 einerseits und gegenüberliegend einteilig an der Führungshülse 17 andererseits ausgebildet. Alternativ können hier auch getrennte Teile eingesetzt werden. Die Werkzeugaufnahme 46 weist ihrerseits am oberen freien Ende eine standardisierte Aufnahme auf, um eine Antriebseinrichtung lösbar daran zu befestigen.

Diese alternative Ausführungsform weist den Vorteil auf, dass in der Werkzeugaufnahme 46 im Bereich der Sacklochbohrung zur Aufnahme des Ende des Schaftes 26 des Werkzeuges 18 ein vergrößerter Durchmesser und somit eine vergrößerte Wandstärke zum Einsetzen eines Haltestiftes 51 ermöglicht wird. Alternativ zu dieser in Figur 2b dargestellten lösbaren Trennstelle 34 kann auch ein Bajonettverschluss vorgesehen sein, der am oberen Ende des Schaftes 26 entsprechend angreift.

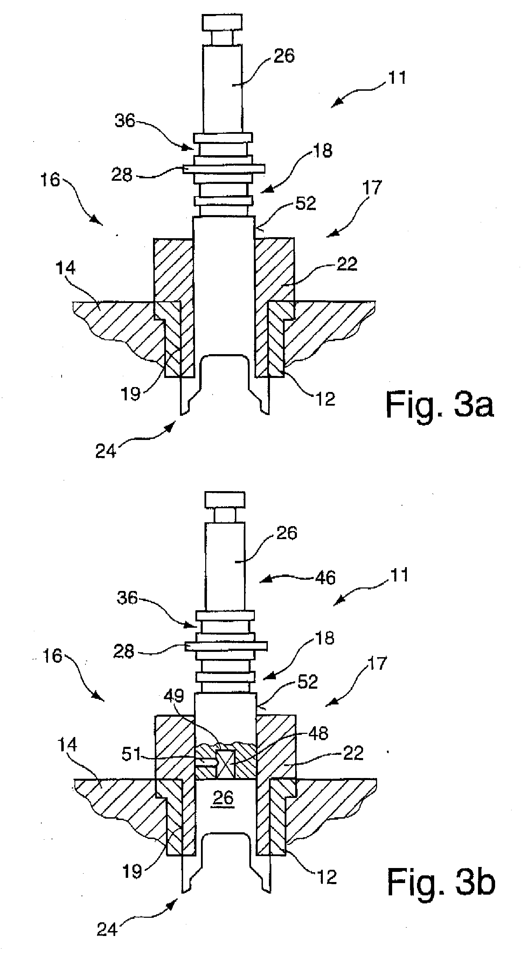

In Figur 3a ist eine schematische Schnittdarstellung einer weiteren alternativen Ausführungsform des Werkzeugsystems 11 mit einer Werkzeugeinheit 16 dargestellt. Diese Werkzeugeinheit 16 umfasst als Werkzeug ein Stanzwerkzeug beziehungsweise ein Hohlbohrwerkzeug, welches zum Stanzen der Schleimhaut 21 verwendet wird, damit der darunter liegende Kiefer frei zugänglich ist. Am Schaft 26 des Werkzeuges 18 sind beispielsweise mehrere Vertiefungen 36 im Abstand zueinander angeordnet. Durch ein entsprechendes Anbringen eines Sicherungselementes 28 kann anwendungsspezifisch ein Tiefenanschlag eingestellt werden.

Das Werkzeug 18 nimmt in Analogie zu den Ausführungsformen gemäß den Figuren 1 und 2 wiederum gesichert die längsverschiebbare Führungshülse 17 auf, so dass bei einem Wechsel des Werkzeuges 18 die komplette Werkzeugeinheit 16 bestehend aus dem Werkzeug 18 und der Führungshülse 17 in die Setzhülse 12 eingesetzt oder aus dieser entfernt wird.

Das in Figur 3b dargestellte Werkzeug 18 ist zweiteilig ausgebildet. Bei dieser Ausführungsform ist beispielsweise am oberen Ende des Schaftes 26 oder Werkzeugkopfes 24 ein vorzugsweise quadratisch ausgebildeter Steckabschnitt als Verdrehsicherung 48 ausgebildet, der in eine komplementäre Vertiefung 49 der Werkzeugaufnahme 46 eingreift, wobei dieser Zapfen der Werkzeugaufnahme 46 wiederum durch einen Haltestift 51 gesichert ist. Die Werkzeugaufnahme 46 kann des Weiteren einen Schaft 26 aufweisen, an dem lösbar das Sicherungselement 28 befestigt ist, um wiederum die Eintauchtiefe für das Werkzeug 18 vorzugeben.

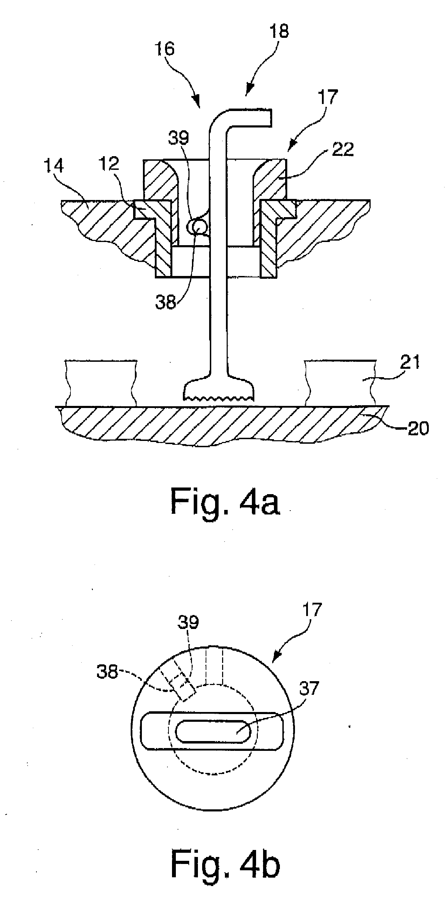

In den Figuren 4a und 4b ist eine weitere Ausführungsform der Werkzeugeinheit 16 dargestellt. Diese umfasst ein Werkzeug 18 zum Spalten des Kiefers 20. Dabei weist die Setzhülse 12 bevorzugt einen Verriegelungsschlitz 38 auf, wobei die Setzhülse 12 bereits lageorientiert in die Bohrschablone 14 eingesetzt wird. Ausgehend von dieser lagerichtig orientierten Setzhülse 12 kann anschließend die Führungshülse 17 eingesetzt werden, welche einen Verriegelungsbolzen 39 umfasst, der in den Verriegelungsschlitz 38 eingreift. Die Führungshülse 17 weist wiederum einen Längsschlitz 37 beziehungsweise Langloch für Schaftdurchmesser auf, so dass das Spaltwerkzeug lageorientiert im Spalt der Führungshülse 17 geführt ist. Im Übrigen gelten die Ausführungen der vorgenannten Figuren.

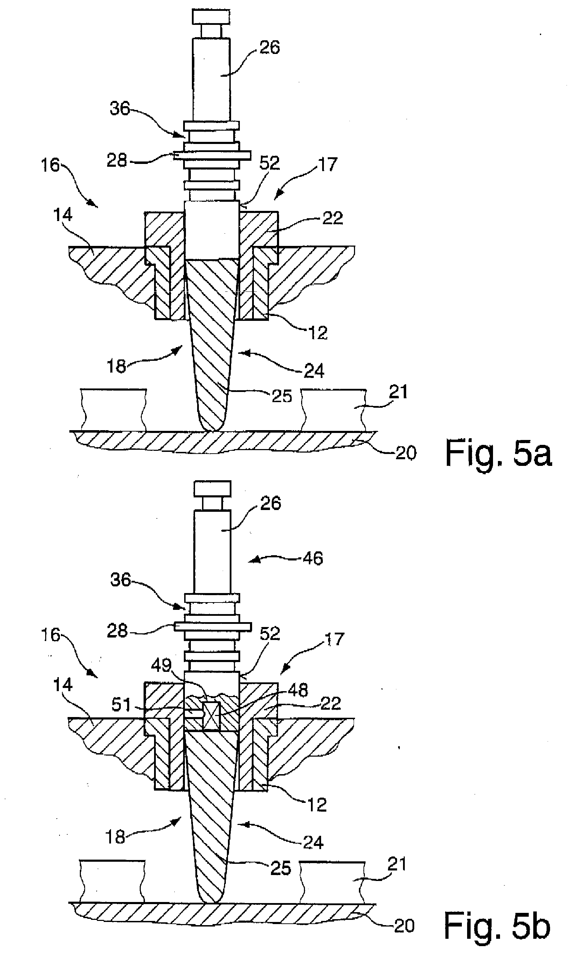

In Figur 5a ist eine weitere Ausführungsform der Werkzeugeinheit 16 dargestellt. Diese Werkzeugeinheit 16 entspricht im Aufbau der Ausführungsform der Werkzeugeinheit in Figur 3a und kann auch eine Trennstelle im Schaft aufweisen. Abweichend hiervon ist anstelle eines Stanzwerkzeuges beziehungsweise eines Hohlbohrwerkzeuges ein Spreizwerkzeug am Schaft 26 angeordnet. Durch ein solches Spreizwerkzeug kann nicht nur eine Spreizung, sondern auch eine Verdichtung des Kieferknochens erfolgen. Die Befestigung des Werkzeuges 18 zur Führungshülse 17 kann beispielsweise dadurch erfolgen, dass ein radial in der Führungshülse 17 eingesetzter und nicht näher dargestellter Bolzen in eine in Achslängsrichtung ausgerichtete langlochförmige Vertiefung am Schaft 26 des Werkzeuges 18 eingreift.

In Figur 5b ist die Werkzeugeinheit 16 gegenüber der Ausführungsform in Figur 5a zweiteilig ausgebildet. Der zweiteilige Aufbau dieser Werkzeugeinheit 16 erfolgt analog zu Figur 3b. Daraus wird auch ersichtlich, dass eine solche Werkzeugaufnahme 46 zur Aufnahme von mehreren unterschiedlichen Werkzeugen 18 geeignet ist. Die Werkzeugaufnahme 46 gemäß den Figuren 3b und 5b weist am Außenumfang jeweils einen Führungsabschnitt 52 auf, durch welchen die Werkzeugeinheit 16 in der Führungshülse 17 entlang der Umfangsfläche 19 der Bohrung geführt wird. Bei der Ausführungsform gemäß Figur 2b liegt die Werkzeugaufnahme 46 außerhalb des Führungsbereiches der Führungshülse 17.

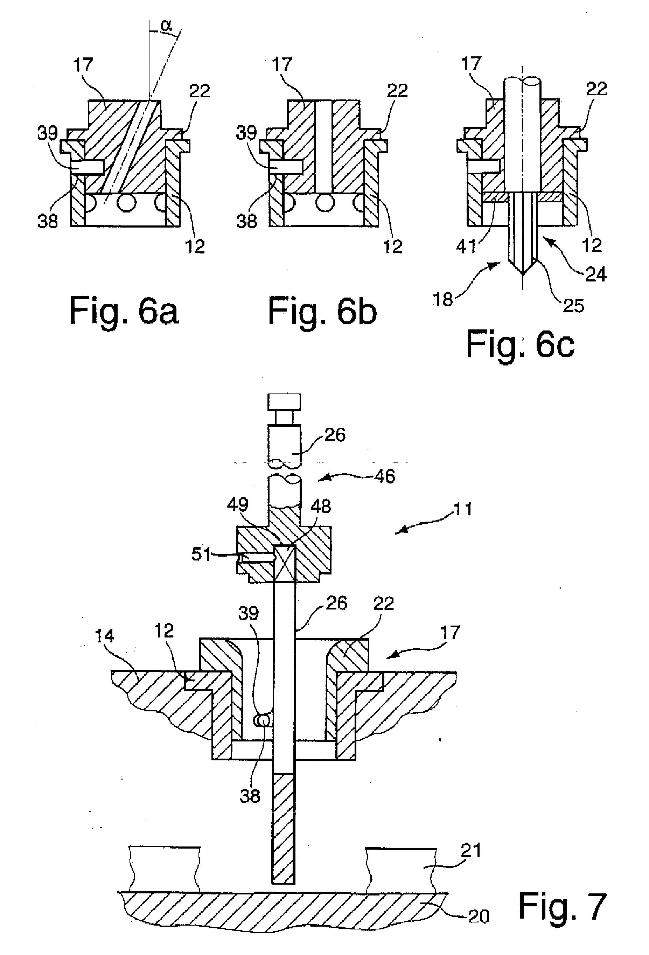

In den Figuren 6a bis c sind schematische Querschnitte von alternativen Ausführungsformen von Führungshülsen 17 dargestellt, welche zur Aufnahme von darin einsetzbaren und entnehmbaren Werkzeugen 18 ausgebildet sind. In Figur 6a umfasst die Führungshülse 17 einen Verriegelungsbolzen 39, der in den Verriegelungsschlitz 38 oder eine Kulisse der Setzhülse 12 eingreift, so dass über die Schulter 22 die Führungshülse 17 in axialer Richtung lagegesichert zur Setzhülse 12 gehalten ist. Über die Umfangsfläche 19 der Führungshülse 17 ist diese radial verkippungsfrei zur Setzhülse 12 gelagert. Dies ist auch bei den vorherigen Ausführungsformen der Fall. Die Führungshülse 17 gemäß Figur 7a weist beispielsweise eine Durchgangsbohrung und einen Winkel α auf, so dass unter diesem Winkel α eine Bohrung, ein Schlitz, eine Spreizung oder Spaltung ermöglicht ist.

Die Ausführungsform der Führungshülse 17 gemäß Figur 6b weicht von der gemäß Figur 6a lediglich dahingehend ab, dass in der Führungshülse 17 eine Längsbohrung entlang der Längsmittelachse der Führungshülse 17 vorgesehen ist, wobei die Längsbohrung in der Draufsicht gesehen als langlochförmige Bohrung, als kreisförmige, dreieckförmige oder jegliche weitere Geometrie umfassende Bohrung ausgebildet sein kann.

In Figur 6c ist eine weitere alternative Ausführungsform der Führungshülse 17 zu den Figuren 6a und b dargestellt. Abweichend hierzu ist an einem zum Implantatbett 13 weisenden Ende der Führungshülse 17 ein Sicherungsring 41 vorgesehen, der wiederum eine Begrenzung beziehungsweise Zustellbegrenzung für das Werkzeug 18 bildet. Dabei ist das Werkzeug 18 bevorzugt mit einem im Durchmesser vergrößerten Schaft 26 gegenüber dem Werkzeugkopf 24 ausgebildet.

In Figur 7 ist eine weitere alternative Ausführungsform des hülsengeführten Werkzeugsystems 11 dargestellt. Die in Figur 2b dargestellte Werkzeugaufnahme 46 ermöglicht beispielsweise auch die Aufnahme eines Fräsers in einer Führungshülse 17, welcher in einem Längsschlitz 37 der Führungshülse 17 geführt ist. Die Führungshülse 17 entspricht der Ausführungsform gemäß den Figuren 4a und b und ist gesichert in der Setzhülse 12 angeordnet. Durch diesen Längsschlitz 37 kann eine Langlochfräsung im Implantatbett 20 erfolgen. Sofern anstelle des Längsschlitzes 37 eine Spaltkontur eingebracht ist, kann eine Fräsung entlang dieser Spaltkontur durchgeführt werden. Die Werkzeugaufnahme 46 kann als eine Tiefenbeschränkung für die Vorschubbewegung des Werkzeuges 18 in das Implantatbett dienen.

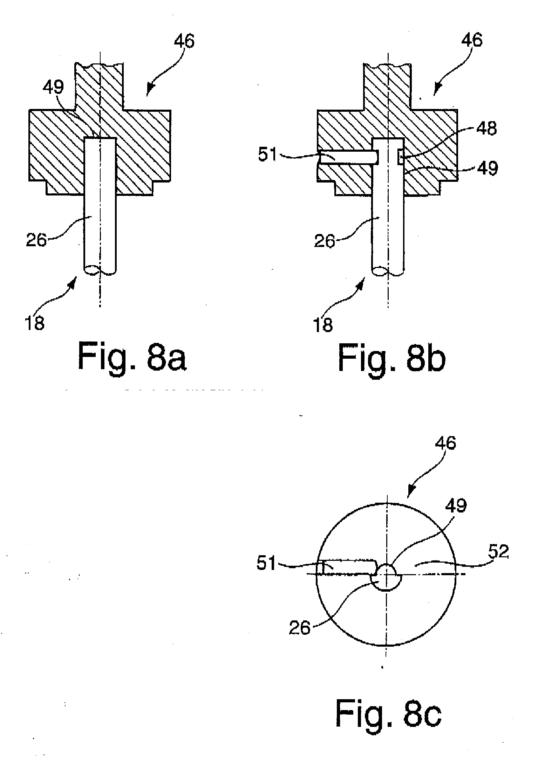

In Figur 8a ist eine schematische Schnittdarstellung einer alternativen Ausführungsform zur Befestigung eines Schaftendes des Werkzeuges 18 in der Werkzeugaufnahme 46 dargestellt. Beispielsweise ist das Schaftende leicht konisch ausgebildet und in eine zylindrische Sacklochbohrung der Werkzeugaufnahme 46 eingesetzt. Durch ein Aufschrumpfen kann eine drehsichere Anordnung und axiale Sicherung geschaffen werden.

In Figur 8b ist eine weitere alternative Ausführungsform dargestellt. Bei dieser Ausführungsform können in dem Schaft 26 eine oder mehrere, entlang einer Mantellinie verteilte, Bohrungen vorgesehen sein, in welche ein Haltestift 51 eingreift, um sowohl eine axiale Sicherung als auch eine Verdrehsicherung zur Werkzeugaufnahme 46 auszubilden.

In Figur 8c ist eine weitere alternative Ausführungsform der Werkzeugaufnahme 46 mit einem darin angeordneten Ende des Schaftes 26 des Werkzeuges 18 in einer Draufsicht dargestellt. Die Werkzeugaufnahme 46 weist eine konturierte Vertiefung 49, insbesondere durch Senkerodierung mit der dargestellten Kontur, auf. Der Schaft 26 ist im oberen Abschnitt im Querschnitt gesehen komplementär zum Einsetzen in die Vertiefung 49 ausgebildet. Durch den Haltestift 51 kann der Schaft 26 axial gesichert werden. Aufgrund der Kontur der Vertiefung 49 ist eine radiale Verdrehsicherung gegeben.

Die vorstehenden Ausführungsbeispiele zur Ausgestaltung einer lösbaren Trennstelle 34 können anwendungsspezifisch an den einzelnen Werkzeugen 18 eingesetzt werden.

## Patentansprüche

1. Hülsengeführtes Werkzeugsystem zum Präparieren eines Implantatbettes (13) zum anschließenden Setzen eines Implantates mit einer Setzhülse (12), welche in eine Bohrschablone (14) befestigbar ist, die zur Implantatsetzung im Mund des Patienten eingesetzt wird, wobei die Setzhülse (12) an der Stelle der Implantatsetzung in der Bohrschablone (14) positionierbar ist, **dadurch gekennzeichnet,**
- **dass** eine Werkzeugeinheit (16) in die Setzhülse (12) einsetzbar ist, welche zumindest aus einem Werkzeug (18) und einer Führungshülse (17) besteht und dass die Führungshülse (17) das Werkzeug (18) längsverschiebbar aufnimmt und daran befestigt ist oder
- **dass** eine Führungshülse (17), welche zur Aufnahme und Führung eines Werkzeuges (18) vorgesehen ist, in die Setzhülse (12) einsetzbar und lagegesichert zu dieser verriegelbar ist sowie anschließend das Werkzeug (18) längsverschiebbar geführt aufnimmt.

2. Werkzeugsystem nach Anspruch 1, **dadurch gekennzeichnet, dass** die Führungshülse (17) am Schaft (26) des Werkzeuges (18) angreift.

3. Werkzeugsystem nach Anspruch 2, **dadurch gekennzeichnet, dass** das Werkzeug (18) einen gegenüber einem Durchmesser des Schaftes (26) größeren Werkzeugkopf (24) und entfernt zum Werkzeugkopf (24) ein am Schaft (26) angeordnetes lösbares Sicherungselement (28) aufweist, wobei dazwischen liegend die Führungshülse (17) längsverschiebbar befestigt ist.

4. Werkzeugsystem nach Anspruch 3, **dadurch gekennzeichnet, dass** zwischen dem Werkzeugkopf (24) und dem Sicherungselement (28) die Führungshülse (17) und eine Federführung (32) mit zumindest einem Federelement (31) und die Federführung (32) zwischen der Führungshülse (17) und dem Sicherungselement (28) angeordnet ist.

5. Werkzeugsystem nach Anspruch 2, **dadurch gekennzeichnet, dass** an dem Schaft (26) des Werkzeuges (24) mehrere im Abstand zueinander angeordnete Vertiefungen (36) zur lösbaren Anordnung des Sicherungselementes (28) vorgesehen sind.

6. Werkzeugsystem nach Anspruch 3, **dadurch gekennzeichnet, dass** zwischen dem Werkzeugkopf (24) und dem lösbaren Sicherungselement (28) zumindest eine lösbare Trennstelle (34) vorgesehen ist.

7. Werkzeugsystem nach Anspruch 2, **dadurch gekennzeichnet, dass** an einem dem Werkzeugkopf (24) gegenüberliegenden Ende oder an einem dem Werkzeugkopf (24) gegenüberliegenden Ende des Schaftes (26) des Werkzeuges (18) eine Werkzeugaufnahme (46) lösbar befestigt ist und eine lösbare Trennstelle (34) bildet.

8. Werkzeugsystem nach Anspruch 6 oder 7, **dadurch gekennzeichnet, dass** die lösbare Trennstelle (34) als Verschraubung ausgebildet ist, eine mit einem Haltestift (51) und zumindest einer Verdrehsicherung (48) ausgebildete Verbindung umfasst oder als eine Aufschrumpfverbindung ausgebildet ist.

9. Werkzeugsystem nach Anspruch 7, **dadurch gekennzeichnet, dass** an der Werkzeugaufnahme (46) und an der Führungshülse (17) jeweils eine Federführung (32) ausgebildet ist.

10. Werkzeugsystem nach Anspruch 1, **dadurch gekennzeichnet, dass** die Führungshülse (17) zur Aufnahme eines Spaltwerkzeuges, Bohr- oder Fräswerkzeuges mit einem Schlitzverschluss zur Setzhülse (12) gesichert ist.

11. Werkzeugsystem nach Anspruch 1, **dadurch gekennzeichnet, dass** die Führungshülse (17) einen radial gegenüber einer an der Setzhülse (12) angreifenden Umfangsfläche (21) hervorstehenden Verriegelungsbolzen (39) aufweist, welcher in eine Kulisse oder einen Verriegelungsschlitz (38) der Setzhülse (12) eingreift.

12. Werkzeugsystem nach Anspruch 1, **dadurch gekennzeichnet, dass** die Führungshülse (17) an einem zum Implantatbett (13) weisenden Ende eine Tiefenbegrenzung, insbesondere einen Sicherungsring (41), aufweist.

13. Werkzeugsystem nach einem der vorhergehenden Ansprüche, **dadurch gekennzeichnet, dass** das Werkzeug (18) der Werkzeugeinheit (16) als Bohr-, Fräs-, Spreiz- oder Spaltwerkzeug ausgebildet ist.

14. Werkzeugsystem nach Anspruch 13, **dadurch gekennzeichnet, dass** das Bohr- oder Fräswerkzeug mit einer innenliegenden Kühlmittelbohrung ausgebildet ist.
